(19) Europäisches Patentamt
European Patent Office
Office européen des brevets

(11) EP 1 269 978 B1

(12) **EUROPÄISCHE PATENTSCHRIFT**

(45) Veröffentlichungstag und Bekanntmachung des Hinweises auf die Patenterteilung:
**24.08.2011 Patentblatt 2011/34**

(51) Int Cl.:
***A61K 8/49*** *(2006.01)* ***A61K 8/31*** *(2006.01)*
***A61Q 19/00*** *(2006.01)*

(21) Anmeldenummer: **02014323.6**

(22) Anmeldetag: **27.06.2002**

(54) **Verwendung von Bis-Resorcinyltriazinderivaten in kosmetischen oder dermatologischen Zubereitungen**

Use of bis-resorcinyltriazine derivatives in cosmetic or dermatological preparations

Utilisation de dérivés de la bis-resorcinyl triazine dans des préparations cosmétiques ou dermatologiques

(84) Benannte Vertragsstaaten:
**AT BE CH CY DE DK ES FI FR GB GR IE IT LI LU MC NL PT SE TR**

(30) Priorität: **27.06.2001 DE 10130963**

(43) Veröffentlichungstag der Anmeldung:
**02.01.2003 Patentblatt 2003/01**

(73) Patentinhaber: **Beiersdorf AG**
**20245 Hamburg (DE)**

(72) Erfinder:
- **Gottwald, Susen**
**22309 Hamburg (DE)**
- **Rapp, Claudius, Dr.**
**20255 Hamburg (DE)**
- **Raschke, Thomas, Dr.**
**25421 Pinneberg (DE)**

(56) Entgegenhaltungen:
**EP-A- 0 949 251** **US-A- 5 962 452**
**US-B1- 6 221 342**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung von Bis-Resorcinyltriazinderivaten zur Verbesserung der Wirksamkeit von lipophilen Wirkstoffen in kosmetischen oder dermatologischen Zubereitungen. Bevorzugt betrifft die vorliegende Erfindung die Verwendung von Bis-Resorcinyltriazinderivaten zur Verbesserung der Wirksamkeit von ungesättigten Hautpflegestoffen, wie beispielsweise Ubichinonen, Retinoiden und Carotinoiden und kosmetische oder dermatologische Zubereitungen, in welchen derartige Wirkstoffe über einen langen Zeitraum stabil gelagert werden können und welche ein gutes Transportmittel für diese Wirkstoffe darstellen.
Kosmetische Hautpflege dient in erster Linie dazu, die natürliche Funktion der Haut als Barriere gegen Umwelteinflüsse (z. B. Schmutz, Chemikalien, Mikroorganismen) und gegen den Verlust von körpereigenen Stoffen (neben Wasser auch natürliche Fette, Elektrolyte etc.) zu stärken oder wiederherzustellen.
Wird diese Funktion gestört, kann es zu verstärkter Resorption toxischer oder allergener Stoffe oder zum Befall von Mikroorganismen und als Folge zu toxischen oder allergischen Hautreaktionen kommen.
Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.
Die chronologische Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:

a) Trockenheit, Rauhigkeit und Ausbildung von Trockenheitsfältchen,
b) Juckreiz und
c) verminderte Rückfettung durch Talgdrüsen (z. B. nach Waschen).

Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgen-7den Strukturschäden- und Funktionsstörungen in der Haut, die über Maß und Qualität der Schäden bei chronologischer Alterung hinausgehen:

d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Die vorliegende Erfindung betrifft insbesondere Produkte zur Pflege der auf natürliche Weise gealterten Haut sowie zur Behandlung der Folgeschäden der Lichtalterung, insbesondere der unter a) bis g) aufgeführten Phänomene, vorzugsweise solche, welche sich durch eine gesteigerte Wirksamkeit auszeichnen.
Produkte zur Pflege gealterter Haut sind an sich bekannt. Sie enthalten z. B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar. Insbesondere betrifft die vorliegende Erfindung kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.
Die vorliegende Erfindung betrifft ferner Antioxidantien, bevorzugt solche, welche in hautpflegenden kosmetischen oder dermatologischen Zubereitungen eingesetzt werden. Insbesondere betrifft die Erfindung auch kosmetische und dermatologische Zubereitungen, solche Antioxidantien enthaltend. In einer bevorzugten Ausführungsform betrifft die vorliegende Erfindung kosmetische und dermatologische Zubereitungen zur Prophylaxe und Behandlung kosmetischer oder dermatologischer Hautveränderungen wie z. B. der Hautalterung, insbesondere der durch oxidative Prozesse hervorgerufenen Hautalterung.
Weiterhin betrifft die vorliegende Erfindung Wirkstoffe und Zubereitungen, solche Wirkstoffe enthaltend, zur kosmetischen und dermatologischen Behandlung oder Prophylaxe erythematöser, entzündlicher, allergischer oder autoimmunreaktiver Erscheinungen, insbesondere Dermatosen.
Die vorliegende Erfindung betrifft in einer weiteren vorteilhaften Ausführungsform Wirkstoffkombinationen und Zubereitungen, die zur Prophylaxe und Behandlung der lichtempfindlichen Haut, insbesondere von Photodermatosen, dienen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereich, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den kosmetischen bzw. dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher auch, kosmetische, dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z. B. A. Voelckel et al, Zentralblatt Haut- und Geschlechtskrankheiten (1989), 156, S.2), angegeben sind.

Erythematöse Hauterscheinungen treten auch als Begleiterscheinungen bei gewissen Hauterkrankungen oder -unregelmäßigkeiten auf. Beispielsweise ist der typische Hautausschlag beim Erscheinungsbild der Akne regelmäßig mehr oder weniger stark gerötet.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können. Solche Prozesse spielen eine wesentliche Rolle bei der Hautalterung.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/ Californien), werden oxidative Schäden der Haut und ihre näheren Ursachen aufgeführt.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der mit der endogenen, chronologischen und exogenen Hautalterung verbundenen Schäden und die Prophylaxe dauerhaft, nachhaltig und ohne das Risiko von Nebenwirkungen sein.

Diesen Übelständen abzuhelfen, war weitere Aufgabe der vorliegenden Erfindung.

Den bei weitem wichtigsten Produkttyp im Bereich der Hautpflegemittel stellen Emulsionen dar. Emulsionen sind disperse Zwei- oder Mehrphasensysteme, wobei kosmetische Emulsionen aus mindestens einer Fettphase (Fette und mineralische Öle, Fettsäureester, Fettalkohole etc.) und mindestens einer Wasserphase (Wasser, Glycerin, Glykole

usw.) bestehen, die mit Hilfe von Emulgatoren in Form feinster Tröpfchen ineinander verteilt werden. Liegt die Ölphase fein verteilt in der Wasserphase vor, so handelt es sich um eine Öl-in-Wasser-Emulsion (O/W-Emulsion, z. B. Milch). Der Grundcharakter einer O/W-Emulsion ist durch das Wasser geprägt, Im Gegensatz zu einer W/O-Emulsion wirkt sie daher weniger fettend auf der Haut, ist eher mattierend und zieht schneller in die Haut ein. O/W-Emulsionen werden aufgrund dieser sensorischen Vorteile für Cremes und Lotionen verwendet, die keinen sichtbaren Fettglanz auf der Haut hinterlassen sollen, wie beispielsweise für Tages- (Gesichts-)cremes und -lotionen.

Der Stand der Technik kennt eine Reihe von verschiedenen effizienten, lipophilen Hautpflegewirkstoffen - wie beispielsweise Ubichinone, Retinoide und Carotinoide - die ungesättigte, aromatische oder benzoide Strukturelemente enthalten, deren Einsatz in kosmetischen und dermatologischen Formulierungen, insbesondere in Formulierungen vom Typ Öl-in-Wasser, sehr wünschenswert ist. Unglücklicherweise sind derartige Substanzen aber häufig sehr instabil, so daß sie insbesondere in wäßrigen Medien schnell zerfallen und auf diese Weise ihre Wirksamkeit verlieren.

Da aber gerade O/W-Formulierungen in diesem Zusammenhang als wäßrige Systeme anzusehen sind, stellt die Einarbeitung von solchen Wirkstoffen in diesen Formulierungstyp bisher ein teilweise erhebliches Problem dar, so daß sich häufig nur schwer eine wirksame Konzentration erreichen läßt.

Aufgabe der vorliegenden Erfindung war es daher insbesondere, die Nachteile des Standes der Technik zu beseitigen und lipophile Hautpflegewirkstoffe, insbesondere Ubichinone, Retinoide und Carotinoide, vor dem Zerfall zu schützen und so ihre Wirksamkeit zu erhöhen, und zwar bevorzugt in kosmetischen oder dermatologischen Zubereitungen. Ferner war Aufgabe, kosmetische oder dermatologische Zubereitungen zu finden, in welchen lipophile Hautpflegewirkstoffe über einen langen Zeitraum stabil gelagert werden können und welche ein gutes Transportmittel für diese Wirkstoffe darstellen.

Es war überraschend und für den Fachmann nicht vorherzusehen, daß die Verwendung von Bis-Resorcinyltriazinderivaten gemäß Anspruch 1 zur Verbesserung der Wirksamkeit von lipophilen Wirkstoffen in kosmetischen oder dermatologischen Zubereitungen, den Nachteilen des Standes der Technik abhelfen.

Es war für den Fachmann nicht vorauszusehen gewesen, daß die Verwendung von Bis-Resorcinyltriazinderivaten gemäß Anspruch 1 lipophile Wirkstoffe besser gegen den Zerfall stabilisieren würde. Es war daher besonders überraschend, daß die erfindungsgemäß verwendeten Bis-Resorcinyltriazinderivate lipophile Wirkstoffe in O/W-Emulsionen ausgezeichnet stabilisieren. Die Stabilität von lipophilen Wirkstoffen in Öl-in-Wasser-Formulierungen läßt sich durch die erfindungsgemäße, Verwendung gegenüber dem Stand der Technik erheblich steigern.

Erfindungsgemäß vorteilhaft zu verwendende Bis-Resorcinyltriazinderivate sind die mit der folgenden Struktur:

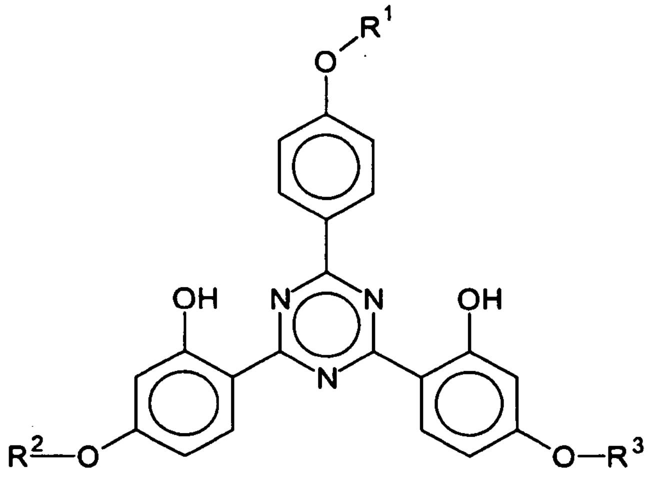

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethylhexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Auch in der Europäischen Offenlegungsschrift 775 698 werden bevorzugt einzusetzende Bis-Resorcinyltriazinderivate beschrieben, deren chemische Struktur durch die generische Formel

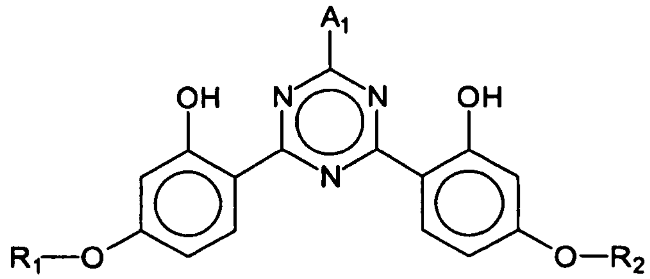

wiedergegeben wird, wobei $R_1$, $R_2$ und $A_1$ verschiedenste organische Reste repräsentieren.

Im Sinne der vorliegenden Erfindung vorteilhaft zu verwenden sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"methyl-propyloxy)-2-hydroxy]-phenyl}-6-(4-methoxyphenyl)-1,3,5-triazin.

Die Menge an Bis-Resorcinyltriazinderivaten (eine oder mehrere Verbindungen) in den Zubereitungen beträgt vorzugsweise 0,05 bis 20 Gew.-%, besonders bevorzugt 0,1 bis 10 Gew.-%, bezogen auf das Gesamtgewicht der Zubereitung.

Vorteilhafte lipophile Wirkstoffe im Sinne der vorliegenden Erfindung sind solche, deren log P-Wert größer als 3,5 ist. P ist der Verteilungskoeffizient, welcher definiert ist als das Verhältnis der Gleichgewichtskonzentrationen einer gelösten Substanz in einem ZweiPhasen-System, welches aus zwei miteinander im wesentlichen nicht mischbaren Lösungsmitteln besteht. Diese beiden Lösungsmittel sind im vorliegenden Fall n-Octanol und Wasser, d. h.

$$P_{ow} = \frac{c_{n\text{-}Octanol}}{c_{Wasser}}$$

Es ist vorteilhaft im Sinne der vorliegenden Erfindung, den oder die lipophile(n) Wirkstoffe aus der Gruppe der Ubichinone und Plastochinone zu wählen.

Ubichinone zeichnen sich durch die Strukturformel

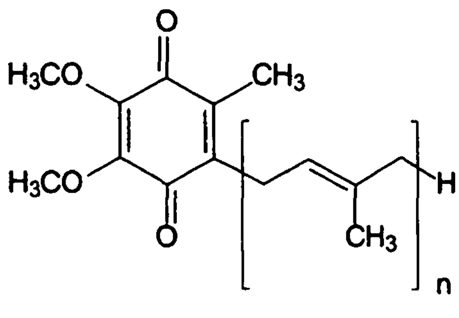

aus und stellen die am weitesten verbreiteten und damit am besten untersuchten Biochinone dar. Ubichinone werden je nach Zahl der in der Seitenkette verknüpften Isopren-Einheiten als Q-1, Q-2, Q-3 usw. oder nach Anzahl der C-Atome als U-5, U-10, U-15 usw. bezeichnet. Sie treten bevorzugt mit bestimmten Kettenlängen auf, z. B. in einigen Mikroorganismen und Hefen mit n=6. Bei den meisten Säugetieren einschließlich des Menschen überwiegt Q10.

Ganz besonders vorteilhaft im Sinne der vorliegenden Erfindung ist Coenzym Q10, welches einen log P-Wert von etwa 15 hat und durch folgende Strukturformel gekennzeichnet ist:

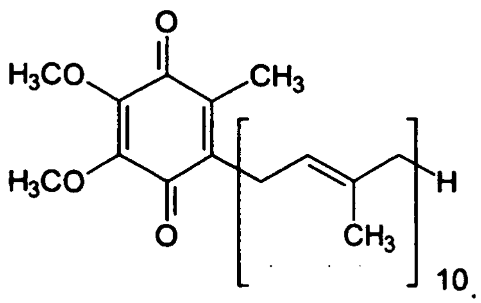

Plastochinone weisen die allgemeine Strukturformel

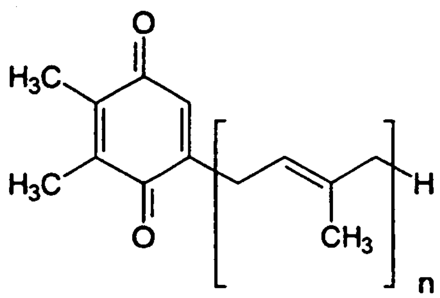

auf. Plastoschinone unterscheiden sich in der Anzahl n der Isopren-Reste und werden endsprechend bezeichnet, z. B. PQ-9 (n=9). Ferner existieren andere Plastochinone mit unterschiedlichen Substituenten am Chinon-Ring.

Es war insbesondere überraschend, daß sich sehr vorteilhafte Zubereitungen gemäß der vorliegenden Erfindung erhalten lassen, wenn der oder die Wirkstoff(e) nur aus der Gruppe der Ubichinone ausgewählt wird/werden.

Weitere erfindungsgemäß vorteilhafte lipophile Wirkstoffe sind Retinoide (Vitamin A-Säure und/oder deren Derivate) bzw. Vitamin A und/oder dessen Derivate. In die Gruppe der erfindungsgemäß vorteilhaften Retinoide werden begrifflich alle kosmetisch und/oder pharmazeutisch unbedenklichen Retinoide, einschließlich des Retinols und seiner Ester, des Retinals sowie der Retinoësäure (Vitamin A-Säure) und deren Ester einbezogen.

Retinol (Vitamin $A_1$, Vitamin A-Alkohol) ist durch die sfolgende Struktur gekennzeichnet:

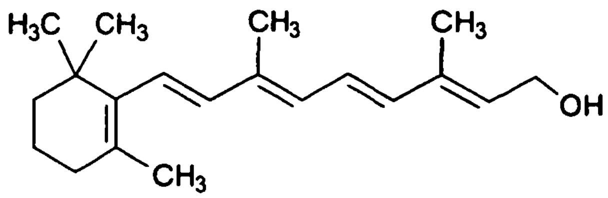

Retinoide sind Verbindungen, die sich vom natürlichen Retinol oder synthetischen Analoga ableiten. Retinoide sind $C_{20}$-Isoprenoide mit einem apolaren cyclischen Rest an einem und einer polaren Gruppe (Carboxy-, Ester-, Amid- oder Alkohol-Gruppe) am anderen Ende.

Besonders vorteilhaft im Sinne der vorliegenden Erfindung sind Retinol (mit einem log P-Wert von etwa 7) und Retinylpalmitat (mit einem log P-Wert von etwa 13).

Es war ferner insbesondere überraschend, daß sich sehr vorteilhafte Zubereitungen gemäß der vorliegenden Erfindung erhalten lassen, wenn der oder die Wirkstoff(e) nur aus der Gruppe der Retinoide ausgewählt wird/werden.

Weitere erfindungsgemäß vorteilhafte lipophile Wirkstoffe sind Carotinoide. "Carotinoid" ist die von Carotin abgeleitete Bezeichnung für Carotine (reine Kohlenwasserstoffe) und Xanthophylle (Sauerstoff-haltige Carotine), deren Grundgerüst aus acht Isopren-Einheiten (Tetraterpene) besteht. Man kann sich die Carotinoide aus zwei $C_{20}$-Isoprenoiden derart zusammengesetzt denken, daß die beiden mittleren Methyl-Gruppen in 1,6-Stellung zueinander stehen (Kopf-Kopf-Verknüpfung); die beiden Mol.-Hälften gehorchen jeweils der Isopren-Regel.

Besonders vorteilhaft im Sinne der vorliegenden Erfindung ist beispielsweise das ß-Carotin, welches sich durch die folgende Strukturformel auszeichnet:

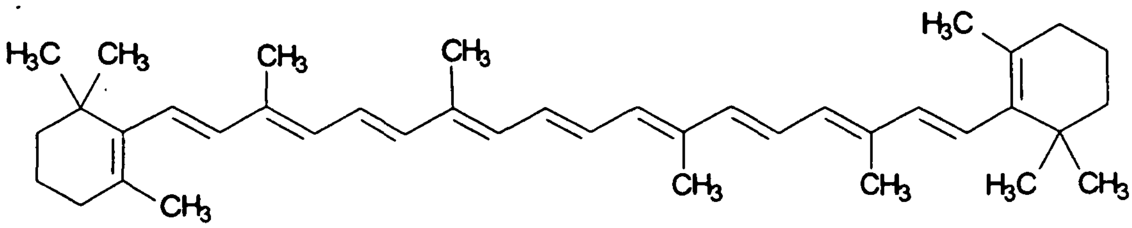

und einen log P-Wert von 15 hat.

Die Menge an lipophilen Wirkstoffen (eine oder mehrere Verbindungen) in den Zubereitungen beträgt vorzugsweise 0,0001 bis 10 Gew.-%, besonders bevorzugt 0,001 bis 1 Gew.-%, bezogen auf das Gesamtgewicht der Zubereitung.

Es ist erfindungsgemäß vorteilhaft, das Gewichtsverhältnis von Bis-Resorcinyltriazinderivaten (eine oder mehrere Verbindungen) zu dem oder den lipophilen Wirkstoffe(n) aus dem Bereich von 1000: 1 bis 1 : 10 zu wählen. Insbesondere bevorzugt ist ein Gewichtsverhältnis von etwa 100 : 1 bis 1 : 2.

Bevorzugt stellen die Zubereitungen gemäß der vorliegenden Erfindung Emulsionen, insbesondere O/W-Emulsionen dar.

Die erfindungsgemäß verwendeten kosmetischen oder dermatologischen Zubereitungen können wie üblich zusammengesetzt sein und zur Behandlung, der Pflege und der Reinigung der Haut und/oder der Haare und als Schminkprodukt in der dekorativen Kosmetik dienen.

Es ist erfindungsgemäß bevorzugt, den kosmetischen oder dermatologischen gemäß der vorliegenden Erfindung Komplexbildner zuzufügen.

Komplexbildner sind an sich bekannte Hilfsstoffe der Kosmetologie bzw. der medizinischen Galenik. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner (Chelatoren) Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom oder -Ion zu Ringen geschlossen. Die Zahl der gebundenen Liganden hängt von der Koordinationszahl des zentralen Metalls ab. Voraussetzung für die Chelatbildung ist, daß die mit dem Metall reagierende Verbindung zwei oder mehr Atomgruppierungen enthält, die als Elektronendonatoren wirken.

Der oder die Komplexbildner können vorteilhaft aus der Gruppe der üblichen Verbindungen gewählt werden, wobei bevorzugt mindestens eine Substanz gewählt wird aus der Gruppe bestehend aus Weinsäure und deren Anionen, Citronensäure und deren Anionen, Iminodibernsteinsäure und deren Anionen, Aminopolycarbonsäuren und deren Anionen (wie beispielsweise Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen) sowie Natrium Chitosan Methylen Phosphonat.

Der oder die Komplexbildner sind erfindungsgemäß vorteilhaft in kosmetischen oder dermatologischen Zubereitungen bevorzugt zu 0,01 Gew.-% bis 15 Gew.-%, bevorzugt zu 0,05 Gew.-% bis 7 Gew.-%, insbesondere bevorzugt zu 0,1 bis 2,0 Gew.-%, bezogen auf das Gesamtgewicht der Zubereitungen, enthalten.

Zur Anwendung werden die kosmetischen und dermatologischen Zubereitungen erfindungsgemäß in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O), eine multiple Emulsionen, beispielsweise vom Typ Wasser-in-Öl-in-Wasser (W/O/W), ein Gel, einen festen Stift, eine Salbe oder auch ein Aerosol darstellen. Erfindungsgemäß besonders vorteilhaft sind kosmetische und dermatologische Zubereitungen in Form von Öl-in-Wasser-Emulsionen mit mindestens einer wäßrigen Phase.

Es ist auch möglich und vorteilhaft im Sinne der vorliegenden Erfindung, Zubereitungen gemäß der vorliegenden Erfindung als wäßrige Systeme bzw. Tensidzubereitungen zur Reinigung der Haut und der Haare zu formulieren.

Die kosmetischen und dermatologischen Zubereitungen gemäß der vorliegenden Erfindung können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Besonders vorteilhafte Zubereitungen gemäß der Erfindung werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. $\alpha$-Carotin, $\beta$-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, $\gamma$-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis $\mu$mol/kg), ferner (Metall)-Chelatoren (z. B. $\alpha$-Hydroxyfettsäuren, Palmitinsäure, Phytinsäure, Lactoferrin), $\alpha$-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. $\gamma$-Linolensäure, Linolsäure, Ölsäure), Folsäure und deren Derivate, Ubichinon und Ubichinol und deren Derivate, Vitamin C und Derivate (z. B. Ascorbylpalmitat, Mg - Ascorbylphosphat, Ascorbylacetat), Tocopherole und Derivate (z. B. Vitamin E - acetat), Vitamin A und Derivate (Vitamin A - palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, $ZnSO_4$) Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Als Emulsionen vorliegende Zubereitungen gemäß der vorliegenden Erfindung enthalten einen oder mehrere Emulgatoren. O/W-Emulgatoren können beispielsweise vorteilhaft gewählt werden aus der Gruppe der polyethoxylierten bzw. polypropoxylierten bzw. polyethoxylierten und polypropoxylierten Produkte, z. B.:

- der Fettalkoholethoxylate
- der ethoxylierten Wollwachsalkohole,
- der Polyethylenglycolether der allgemeinen Formel $R\text{-}O\text{-}(\text{-}CH_2\text{-}CH_2\text{-}O\text{-})_n\text{-}R'$,
- der Fettsäureethoxylate der allgemeinen Formel $R\text{-}COO\text{-}(\text{-}CH_2\text{-}CH_2\text{-}O\text{-})_n\text{-}H$,
- der veretherten Fettsäureethoxylate der allgemeinen Formel $R\text{-}COO\text{-}(\text{-}CH_2\text{-}CH_2\text{-}O\text{-})_n\text{-}R'$,
- der veresterten Fettsäureethoxylate der allgemeinen Formel $R\text{-}COO\text{-}(\text{-}CH_2\text{-}CH_2\text{-}O\text{-})_n\text{-}C(O)\text{-}R'$,
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel $R\text{-}O\text{-}(\text{-}CH_2\text{-}CH_2\text{-}O\text{-})_n\text{-}CH_2\text{-}COOH$ nd n eine Zahl von 5 bis 30 darstellen,
- der Polyoxyethylensorbitolfettsäureester,
- der Alkylethersulfate der allgemeinen Formel $R\text{-}O\text{-}(\text{-}CH_2\text{-}CH_2\text{-}O\text{-})_n\text{-}SO_3\text{-}H$
- der Fettalkoholpropoxylate der allgemeinen Formel $R\text{-}O\text{-}(\text{-}CH_2\text{-}CH(CH_3\text{-}O\text{-})_n\text{-}H$,
- der Polypropylenglycolether der allgemeinen Formel $R\text{-}O\text{-}(\text{-}CH_2\text{-}CH(CH_3)\text{-}O\text{-})_n\text{-}R'$,
- der propoxylierten Wollwachsalkohole,
- der veretherten Fettsäurepropoxylate $R\text{-}COO\text{-}(\text{-}CH_2\text{-}CH(CH_3)\text{-}O\text{-})_n\text{-}R'$,

- der veresterten Fettsäurepropoxylate der allgemeinen Formel R-COO-(-$CH_2$-CH($CH_3$)-O-)$_n$-C(O)-R',
- der Fettsäurepropoxylate der allgemeinen Formel R-COO-(-$CH_2$-CH($CH_3$)-O-)$_n$-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel R-O-(-$CH_2$-CH($CH_3$)O-$_n$$CH_2$-COOH
- der Alkylethersulfate bzw. die diesen Sulfaten zugrundeliegenden Säuren der allgemeinen Formel R-O-(-$CH_2$-CH($CH_3$)-O-)$_n$-$SO_3$-H
- der Fettalkoholethoxylate/propoxylate der allgemeinen Formel R-O-$X_n$-$Y_m$-H,
- der Polypropylenglycolether der allgemeinen Formel R-O-$X_n$-$Y_m$-R',
- der veretherten Fettsäurepropoxylate der allgemeinen Formel R-COO-$X_n$-$Y_m$ R',
- der Fettsäureethoxylate/propoxylate der allgemeinen Formel R-COO-$X_n$-$Y_m$-H,.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 bis 18, ganz besonders vorteilhaft mit mit HLB-Werten von 14,5 bis 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:

Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-1 7), Polyethylenglycol-(18)stearylether (Steareth-18), Polyethylenglycol(19)stearylether (Steareth-19), Polyethylenglycol(20)stearylether (Steareth-20),

Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19-), Polyethylenglycol(20) isostearylether (Isosteareth-20),

Polyethylenglycol(13)cetylether (Ceteth-13), Polyethylenglycol(14)cetylether (Ceteth-14), Polyethylenglycol (15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),

Polyethylenglycol(13)isocetylether (Isoceteth-13), Polyethylenglycol(14)isocetylether (isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol(19)isocetylether (Isoceteth-19), Polyethylenglycol(20)isocetylether (Isoceteth-20),

Polyethylenglycol(12)oleylether (Oleth-12), Polyethylenglycol(13)oleylether (Oleth-13), Polyethylenglycol(14) oleylether (Oleth-14), Polyethylenglycol(15)oleylether (Oleth-15),

Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:

Polyethylenglycol(20)stearat, Polyethylenglycol(21)stearat, Polyethylenglycol(22)stearat, Polyethylenglycol (23)stearat, Polyethylenglycol(24)stearat, Polyethylenglycol(25)stearat, Polyethylenglycol(12)isostearat, Polyethylenglycol(13)isostearat, Polyethylenglycol(14)-isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)-isostearat, Polyethylenglycol(23)isostearat, Polyethylenglycol(24)isostearat, Polyethylenglycol(25)isostearat,

Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20) glyceryllaurat, Polyethylenglycol(21)glyceryllaurat, Polyethylenglycol-(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Als Emulgatoren eignen sich besonders Glycerylstearatcitrat, Glycerylstearat, PEG-Ester der Stearinsäure sowie die Kombination Glycerylstearat/Stearinsäure.

Erfindungsgemäß verwendete Gele enthalten üblicherweise Alkohole niedriger C-Zahl, z. B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z. B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z. B. Paraffinöle, Ricinusöl, Isopropylmyristat), halbfeste Bestandteile (z. B. Vaseline, Lanolin), feste Bestandteile (z. B. Bienenwachs, Ceresin und Mikrokristalline Wachse bzw. Ozokerit) sowie hochschmelzende Wachse (z. B. Carnaubawachs, Candelillawachs)

Die Lipidphase der Zubereitungen gemäß der vorliegenden Erfindung kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase wird im Sinne der vorliegenden Erfindung ferner vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkylbenzoat und 2-Ethylhexylisostearat, Mischungen aus $C_{12-15}$-Alkylbenzoat und Isotridecylisononanoat sowie Mischungen aus $C_{12-15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der Zubereitungen gemäß der vorliegenden Erfindung enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden. Auch Bariumsulfat ist vorteilhaft im Sinne der vorliegenden Erfindung.

Solche Pigmente können im Sinne der vorliegenden Erfindung vorteilhaft oberflächlich behandelt ("gecoatet") sein, wobei beispielsweise ein amphiphileroder hydrophober Charakter gebildet werden bzw. erhalten bleiben soll. Diese Oberflächenbehandlung kann darin bestehen, daß die Pigmente nach an sich bekannten Verfahren mit einer dünnen hydrophoben Schicht versehen werden.

Erfindungsgemäß vorteilhaft sind z. B. Titandioxidpigmente, die mit Octylsilanol beschichtet sind. Geeignete Titandioxidpartikel sind unter der Handelsbezeichnung T805 bei der Firma Degussa erhältlich. Besonders vorteilhaft sind ferner mit Aluminiumstearat beschichtete $TiO_2$-Pigmente, z. B. die unter der Handelsbezeichnung MT 100 T bei der Firma TAYCA erhältlichen. Weitere vorteilhafte Titandioxidpigmente sind die unter den Handelsbezeichnungen MT

100 TV, MT 100 Z und MT 100 AQ bei der Firma TAYCA erhältlichen.

Eine weitere vorteilhafte Beschichtung der anorganische Pigmente besteht aus Dimethylpolysiloxan (auch: Dimethicon), einem Gemisch vollmethylierter, linearer Siloxanpolymere, die endständig mit Trimethylsiloxy-Einheiten blockiert sind. Besonders vorteilhaft im Sinne der vorliegenden Erfindung sind Zinkoxid-Pigmente, die auf diese Weise beschichtet werden.

Vorteilhaft ist ferner eine Beschichtung der anorganischen Pigmente mit einem Gemisch aus Dimethylpolysiloxan, insbesondere Dimethylpolysiloxan mit einer durchschnittlichen Kettenlänge von 200 bis 350 Dimethylsiloxan-Einheiten, und Silicagel, welches auch als Simethicone bezeichnet wird. Es ist insbesondere von Vorteil, wenn die anorganischen Pigmente zusätzlich mit Aluminiumhydroxid bzw. Aluminiumoxidhydrat (auch: Alumina, CAS-Nr.: 1333-84-2) beschichtet sind. Besonders vorteilhaft sind Titandioxide, die mit Simethicone und Alumina beschichtet sind, wobei die Beschichtung auch Wasser enthalten kann. Ein Beispiel hierfür ist das unter dem Handelsnamen Eusolex T2000 bei der Firma Merck erhältliche Titandioxid.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel

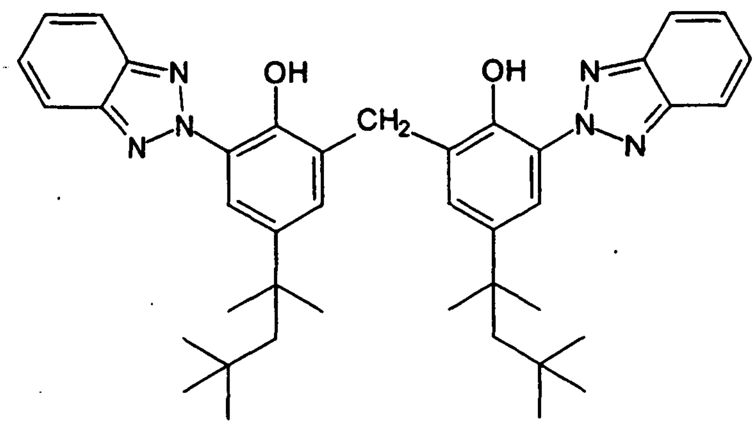

gekennzeichnet ist und unter der Handelsbezeichnung Tinosorb® M bei der CIBA-Chemikalien GmbH erhältlich ist.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure

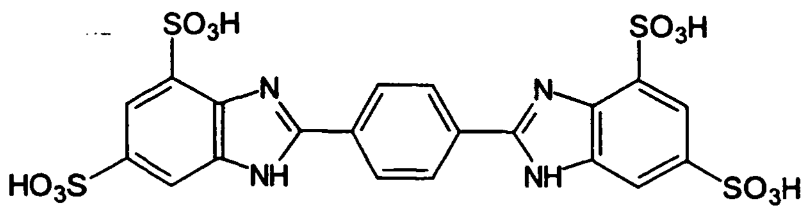

und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz

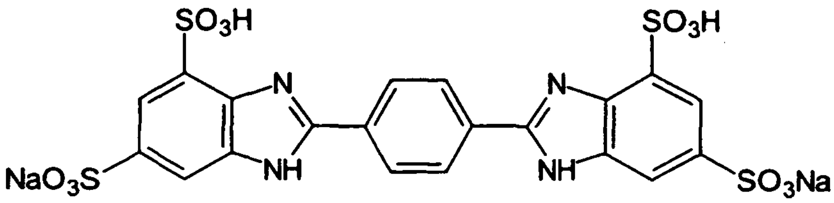

mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird und sich durch die folgende Struktur auszeichnet:

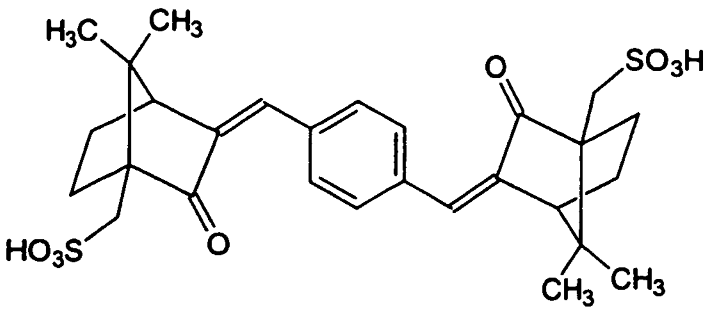

Vorteilhafte UV-Filtersubstanzen im Sinne der vorliegenden Erfindung sind ferner sogenannte Breitbandfilter, d. h. Filtersubstanzen, die sowohl UV-A- als auch UV-B-Strahlung absorbieren.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

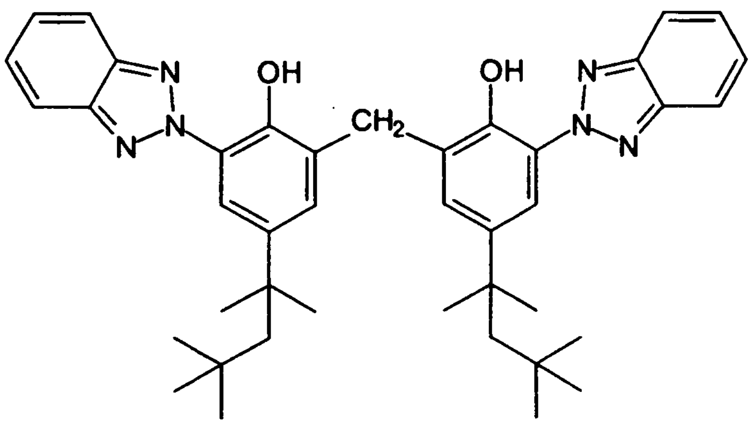

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

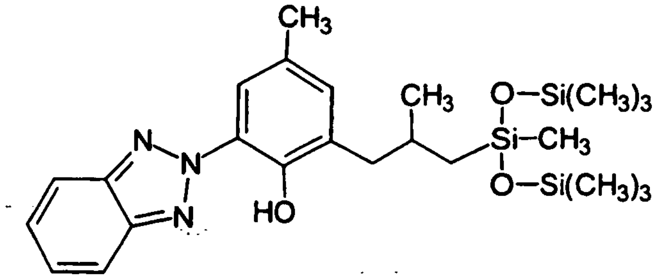

gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- sowie an Polymere gebundene UV-Filter.

Vorteilhafte wasserlösliche UV-B- und/oder Breitband-Filtersubstanzen sind z. B.:

- Salze der 2-Phenylbenzimidazol-5-sulfonsäure, wie ihr Natrium-, Kalium- oder ihr Triethanolammonium-Salz, sowie die Sulfonsäure selbst;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

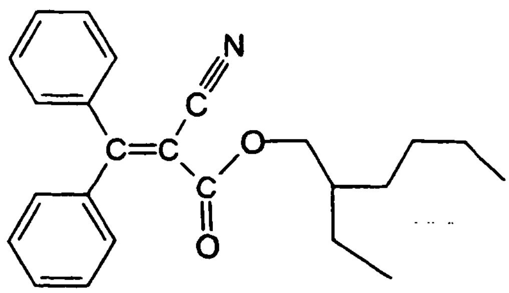

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Ferner kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-Isopropylbenzylsalicylat, 2-Ethylhexylsalicylat (= Octylsalicylat), Homomenthylsalicylat.

Besonders vorteilhafte Zubereitungen im Sinne der vorliegenden Erfindung, die sich durch einen hohen bzw. sehr hohen UV-A-Schutz auszeichnen, enthalten bevorzugt zusätzlich zu einem oder mehreren erfindungsgemäßen Bis-Resorcinyltriazinderivaten mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze, jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.Gegenstand der Erfindung ist auch die Verwendung einer Kombination der erfindungsgemäß verwendeten Zubereitungen mit mindestens einem UV-A-Filter und/oder mindestens einem UV-B-Filter als Antioxidans bzw. die Verwendung einer Kombination der erfindungsgemäß verwendeten Zubereitungen mit mindestens einem UV-A-Filter und/oder mindestens einem UV-B-Filter als Antioxidans in einer kosmetischen oder dermatologischen Formulierung.

Weitere vorteilhafte kosmetische und dermatologische Zubereitungen gemäß der vorliegenden Erfindung sind z. B. Formulierungen zum Schutz der Haare vor UV-Strahlen, Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, Zubereitungen zum Fönen oder Einlegen der Haare, Zubereitungen zum Färben oder Entfärben, Frisier- und/oder Behandlungslotionen, Haarlacke oder auch Dauerwellmittel.

Derartige Zubereitungen können beispielsweise in Form von wäßrigen Lösungen, Emulsionen, Gelen, Schäumen oder dergleichen vorliegen und enthalten vorteilhaft Wirkstoffe und Hilfsstoffe, wie sie üblicherweise für diesen Typ von Zubereitungen zur Haarpflege und Haarbehandlung verwendet werden, wie z. B. Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Diese kosmetischen oder dermatologischen Zubereitungen können auch Schäume bzw. schäumbare Formulierungen bzw. Aerosole mit den üblicherweise dafür verwendeten Hilfsmitteln darstellen. Als Treibmittel für solche, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Auch kosmetische Reinigungszubereitungen für die Haut sind vorteilhaft im Sinne der vorliegenden Erfindung und können z. B. in flüssiger oder fester Form vorliegen. Sie enthalten neben erfindungsgemäß verwendeten Wirkstoffen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Reinigungszubereitungen gemäß der vorliegenden Erfindung enthalten außer den vorgenannten Tensiden vorteilhaft auch Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

**<u>Beispiele:</u>**

O/W-Cremes

| **Beispiel Nummer** | **1** | **4** | **5** | **7** | **9** | **10** |
|---|---|---|---|---|---|---|
| Glycerylstearatcitrat | 2 | 2 | | | | |
| Glycerylsterat, selbstemulgierend | | | 2 | | | |
| PEG-40-Stearat | | | 1 | | | |

(fortgesetzt)

| Beispiel Nummer | 1 | 4 | 5 | 7 | 9 | 10 |
|---|---|---|---|---|---|---|
| PEG-100-Stearat | | | | | | |
| Polyglyceryl-3-Diisostearat | | | | | | |
| Polyglyceryl-3-Methylglucosedistearat | | | | 3 | | |
| Sorbitanstearat | | | | 1 | | |
| Polyethylenglycol(21)stearylether (Steareth-21) | | | | | | |
| Polyethylenglycol(2)stearylether (Steareth-2) | | | | | | |
| Cetearylglucosid | | | | | 2 | |
| Stearinsäure | | | | | | 2,5 |
| Myristylmyristat | 1 | | 1 | | 1 | |
| Behenylalkohol | | | | 1 | | 2 |
| Stearylalcohol | 2 | | | | 5 | |
| Cetearylalkohol | | 4 | 2 | | | 1 |
| Cetylalcohol | 1 | | | 1 | | |
| Hydrierte Cocosfettglyceride (Hydrogenated Coco Glycerides) | 2 | | | | | 1 |
| Sheabutter | | 1 | | | | |
| C12-15 Alkylbenzoat | | | 3 | 2 | 2 | |
| Butylenglycol Dicaprylat/Dicaprat | 1 | 1 | | | | 2 |
| Caprylic/Capric Triglyceride | | 2 | | 1 | | |
| Hydriertes Polydecen | | | | | 1 | |
| Ethylhexylkokosfettsäureester | 3 | | | | | 2 |
| Octyldodecanol | | | | | | |
| Mineralöl | | | | | | |
| Vaseline | 4 | | | | | |
| Octamethyltetrasiloxan (Cyclomethicon) | | 5 | 3 | 3 | | |
| Dimethylpolysiloxan (Dimethicon) | | | 1 | | | 1 |
| Dicaprylylether | 1 | | | | | |
| Dicarprylylcarbonat | | 1 | | 2 | | 4 |
| Polydecen | | | | | 5 | |
| $TiO_2$ | | | 1 | | | 1 |
| Ethylhexylmethoxycinnamat | 3 | | 2 | 3 | 5 | 3 |
| Ethylhexyltriazon | | | | 2 | | |
| Diethylhexylbutamidotriazon | | | | | | |
| Butylmethoxydibenzoylmethan | | | | | | |
| Bis-Ethylhexyloxyphenol-methoxyphenyltriazine | 1 | 3 | 1 | 1 | 2 | 2 |
| Ubichinon (Q10) | 0,05 | | 0.05 | 0,1 | | 0,1 |
| ß-Carotin | | 0,1 | | | | |
| $\alpha$-Glucosylrutin | | 0,1 | | | | |

(fortgesetzt)

| Beispiel Nummer | 1 | 4 | 5 | 7 | 9 | 10 |
|---|---|---|---|---|---|---|
| Citronensäure, Natriumsalz | | | | | | |
| Weinsäure, Natriumsalz | | | | | | |
| Phytinsäure | | | 0,1 | | | |
| Ascorbinsäure | | | | | | |
| Retinol | | | | | 0,1 | |
| Hydroxypropyl-ß-Cyclodextrin | | | 0,5 | | | |
| Lactoferrin | | | | | . | 0,05 |
| Trinatrium EDTA | | | | | 0,2 | |
| Iminodisuccinat | 0,2 | 0,3 | | | | |
| Phenoxyethanol | 0,3 | 0,2 | | 0,4 | | 0,3 |
| p-Hydroxybenzoesäurealkylester (Paraben) | 0,6 | 0,3 | 0,3 | | 0,4 | 0,6 |
| Hexamidindiisethionat | | | 0,04 | | | |
| Diazolidinylharnstoff | 0,25 | 0,2 | | 0,2 | 0,1 | |
| 1,3-Dimethylol-5,5-dimethylhydantoin (DMDM Hydantoin) | | | | | | |
| Iodopropynylbutylcarbamat | 0,1 | | 3 | | | |
| Ethanol denaturiert | 1 | | | | 8 3 | |
| 2-Ethylhexylglycerinether (Octoxyglycerin) | | | | | | |
| Xanthan Gummi | 0,6 | | | 0,1 | | |
| Polyacrylsäure (Carbomer) | 0,05 | | 0,1 | | | |
| Polyacrylamid | | | | 0,2 | | |
| Glycerin | 5 | 7,5 | 5 | 5 | 4 | 5 |
| Butylenglycol | 2 | | 2 | | 2 | |
| wasser- und/oder öllösliche Farbstoffe | 0,05 | | | | | 0,1 |
| Füllstoffe (Distärkephosphat, $SiO_2$, Talkum, Aluminiumstearat) | | 0,5 | | | 5 | 1 |
| Parfüm | q.s. | q.s. | q.s. | q.s. | q.s. | q.s. |
| Wasser | Ad 100 | Ad 100 | Ad 100 | Ad 100 | Ad 100 | Ad 100 |

**Beispiel : Hydrodispersion/Gelcreme**

| | |
|---|---|
| Cetearylalkohol | 2 |
| Sheabutter | 1 |
| Caprylic/Capric Triglyceride | 2 |
| Octyldodecanol | 1 |
| Octamethyltetrasiloxan (Cyclomethicon) | 5 |

(fortgesetzt)

| | |
|---|---|
| Dimethylpolysiloxan (Dimethicon) | 1 |
| Polydecen | 2 |
| Ethylhexylmethoxycinnamat | 5 |
| Bis-Ethylhexyloxyphenolmethoxyphenyltriazine | 1 |
| Ubichinon (Q10) | 0,1 |
| Iminodisuccinat | 0,2 |
| Phenoxyethanol | 0,3 |
| p-Hydroxybenzoesäurealkyleste (Paraben) | 0,4 |
| Polyacrylsäure (Carbomer) | 0,1 |
| Vernetztes Alkylacrylat (Alkylacrylate Cosspolymer) | 0,2 |
| Glycerin | 5 |
| Parfüm | q.s. |
| Wasser | Ad 100 |

**Beispielrezepturen Foundations**

| **Inhaltsstoffe** | **2** | **3** |
|---|---|---|
| PEG-30 Stearate | | |
| Gylceryl Stearate | | |
| Ceteareth-20 | | |
| Polyglyceryl-3 Methylglucose Stearate | | 3,5 |
| Sorbitan Stearate | | 1,5 |
| Stearinsäure | | |
| Glyceryl Stearate Citrate | 3,5 | |
| Cetyl Alcohol | 0,75 | 1,0 |
| Lecithin | | |
| Veegum K = Mg-Al-Silicate | | |
| Xanthan Gum | | |
| Carbomer | 0,2 | |
| Hydroxyethylcellulose | | 0,1 |
| Caprylic / Capric Triglyceride | 2 | 3 |
| Dicaprylylether | 2 | 3 |
| Octyldodecanol | | 3 |
| Dimethicone | | 3 |
| Cyclomethicone | 4 | 3 |
| C12-C15 Alkyl Benzoate | | |
| Cetearyl Octanoate | | |
| Squalane | | |

(fortgesetzt)

| Inhaltsstoffe | 2 | 3 |
|---|---|---|
| Isopropyl Palmitate | | |
| PPG -15 Stearylether | 3 | |
| Hydrogenated Coco-Glycerides | | |
| Stearyl Dimethicone | | |
| Acetylated Lanolin Oil | 0,2 | |
| Octyl Methoxy Cinnamate | 2 | |
| $TiO_2$ | 2 | |
| Ethylhexyltriazon | | |
| Bis-Ethylhexyloxyphenol-methoxyphenyltriazine | 1 | 2 |
| Ubichinon (Q10) | 0.05 | 0.02 |
| Retinylpalmitat | | |
| Silikon | 0,8 | |
| Nylon-12 | | 5 |
| Lauroyl Lysine | | 0,5 |
| Kaolin | | 1 |
| Sodium Starch Octenylsuccinate | 1 | |
| Eisenoxide | 2,4 | 2,6 |
| Titandioxid | 5,6 | 4,5 |
| Interferenz Pigmente | | |
| Pigment Low Lustre | | |
| ZnO | 1 | |
| Polymethylsilsesquioxane (Tospearl 2000B) | 2 | |
| EDTA | 0,6 | 1 |
| Glycerin | 5 | 5 |
| Phenoxyethanol und Parabene (Phenonip) | 0,5 | |
| Imidazonidinyl Urea | 0,3 | |
| Neutralisationsmittel | q.s. | q.s. |
| Parfum, Antioxidans | q.s. | q.s. |
| Wasser | ad 100 | ad 100 |

**Patentansprüche**

1. Verwendung von Verbindungen gewählt aus der Gruppe der Verbindungen

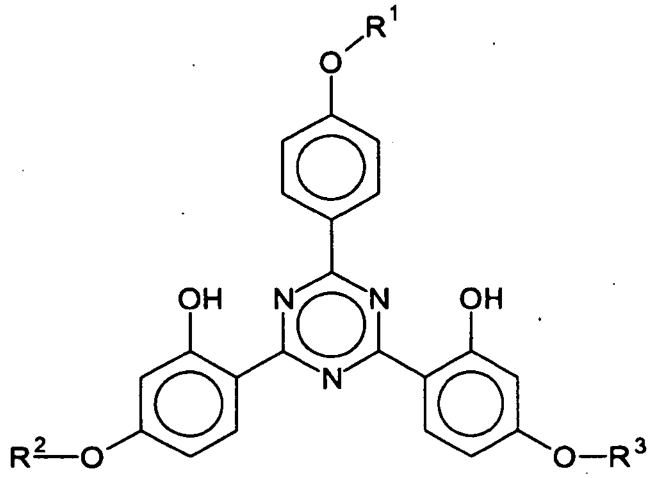

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen, zur Verbesserung der Wirksamkeit von lipophilen Wirkstoffen in kosmetischen oder dermatologischen Zubereitungen.

2. Verwendung nach Anspruch 1, **dadurch gekennzeichnet, daß** die lipophilen Wirkstoffe aus der Gruppe der Wirkstoffe gewählt werden, welche einen log P-Wert haben, der größer als 3,5 ist.

3. Verwendung nach Anspruch 1, **dadurch gekennzeichnet, daß** die lipophilen Wirkstoffe (eine oder mehrere Verbindungen) aus den Gruppen Ubichinone, Retinoide und/oder Carotinoide gewählt werden.

4. Verwendung nach einem der vorhergehenden Ansprüche, **dadurch gekennzeichnet, daß** die Menge an lipophilen Wirkstoffen (eine oder mehrere Verbindungen) in den Zubereitungen aus dem Bereich von 0,0001 bis 1 Gew.-% gewählt wird, bezogen auf das Gesamtgewicht der Zubereitung.

5. Verwendung nach einem der vorhergehenden Ansprüche, **dadurch gekennzeichnet, daß** die Menge an Bis-Resorcinyltriazinderivaten (eine oder mehrere Verbindungen) in den Zubereitungen aus dem Bereich von 0,1 bis 10 Gew.-% gewählt wird, bezogen auf das Gesamtgewicht der Zubereitung.

6. Verwendung nach einem der vorhergehenden Ansprüche, **dadurch gekennzeichnet, daß** das Gewichtsverhältnis von Bis-Resorcinyltriazinderivaten (eine oder mehrere Verbindungen) zu dem oder den lipophilen Wirkstoffe(n) aus dem Bereich von 1000 : 1 bis 1 : 10 gewählt wird.

7. Verwendung nach einem der vorhergehenden Ansprüche, **dadurch gekennzeichnet, dass** als Verbindungen gewählt aus der Gruppe der Verbindungen

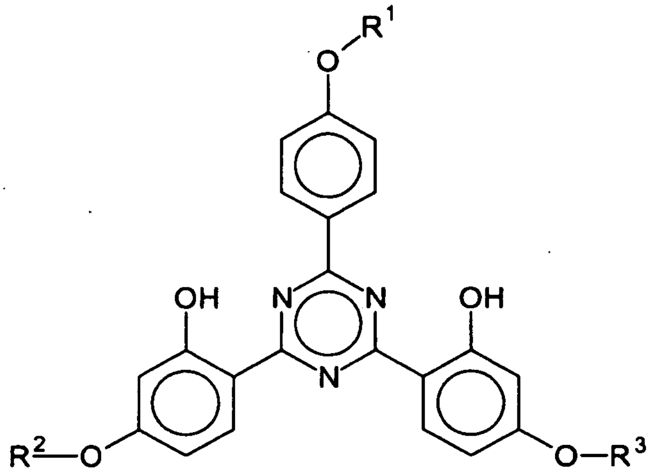

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen, eine oder mehrere Verbindungen gewählt aus der Gruppe der Verbindungen 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin eingesetzt werden.

## Claims

1. Use of compounds selected from the group of the compounds

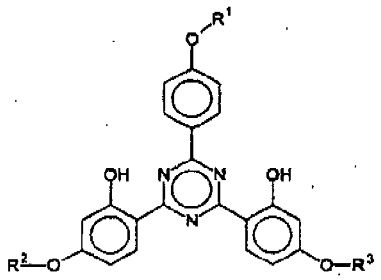

where $R^1$, $R^2$ and $R^3$, independently of one another, are selected from the group of the branched and unbranched alkyl groups having 1 to 10 carbon atoms and/or are an individual hydrogen atom, for improving the effectiveness of lipophilic active ingredients in cosmetic or dermatological preparations.

2. Use according to Claim 1, **characterized in that** the lipophilic active ingredients are selected from the group of the active ingredients which have a log P value which is greater than 3.5.

3. Use according to Claim 1, **characterized in that** the lipophilic active ingredients (one or more compounds) are selected from the groups ubiquinones, retinoids and/or carotenoids.

4. Use according to one of the preceding claims, **characterized in that** the amount of lipophilic active ingredients (one or more compounds) in the preparations is selected from the range from 0.0001 to 1% by weight, based on the total

weight of the preparation.

**5.** Use according to one of the preceding claims, **characterized in that** the amount of bis-resorcinyltriazine derivatives (one or more compounds) in the preparations is selected from the range from 0.1 to 10% by weight, based on the total weight of the preparation.

**6.** Use according to one of the preceding claims, **characterized in that** the weight ratio of bis-resorcinyltriazine derivatives (one or more compounds) to the lipophilic active ingredient(s) is selected from the range from 1000:1 1 to 1:10.

**7.** Use according to one of the preceding claims, **characterized in that** the compounds selected from the group of the compounds

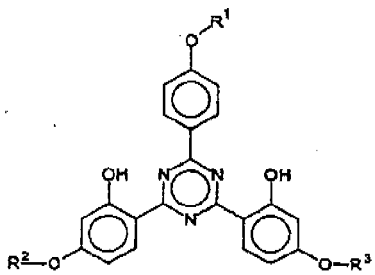

where $R^1$, $R^2$ and $R^3$, independently of one another, are selected from the group of the branched and unbranched alkyl group having 1 to 10 carbon atoms and/or are an individual hydrogen atom, that are used are one or more compounds selected from the group of the compounds 2,4-bis{[4-(3-sulphonato)-2-hydroxypropyloxy)-2-hydroxy]phenyl}-6-(4-methoxyphenyl)-1,3,5-triazine sodium salt, 2,4-bis{[4-(3-(2-propyloxy)-2-hydroxypropyloxy)-2-hydroxy]phenyl}-6,(4-methoxyphenyl)-1,3,5-triazine, 2,4-bis{[4-(2-ethylhexyloxy)-2-hydroxy]phenyl}-6-[4-(2-methoxyethylcarboxyl)phenylamino]-1,3,5-triazine, 2,4-bis{[4-(3-(2-propyloxy)-2-hydroxypropyloxy)-2-hydroxy]phenyl}-6-[4-(2-ethylcarboxyl)phenylamino]-1,3,5-triazine, 2,4-bis{[4-(2-ethylhexyloxy)-2-hydroxy]phenyl}-6-(1-methylpyrrol-2-yl)-1,3,5-triazine, 2,4-bis{[4-tris(trimethylsiloxysilylpropyloxy)-2-hydroxy]phenyl}-6-(4-methoxyphenyl)-1,3,5-triazine, 2,4-bis{[4-(2"-methylpropenyloxy)-2-hydroxy]phenyl}-6-(4-methoxyphenyl)-1,3,5-triazine and 2,4-bis{[4-(1',1',1',3',5',5',5'-heptamethylsiloxy-2"-methylpropyloxy)-2-hydroxy]phenyl}-6-(4-methoxyphenyl)-1,3,5-triazine.

## Revendications

**1.** Utilisation de composés choisis dans le groupe des composés

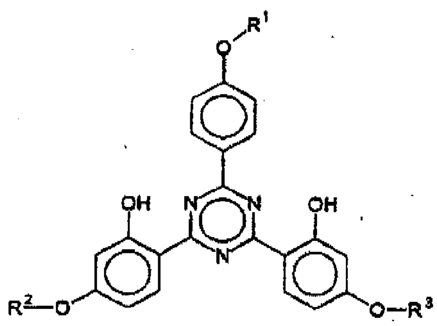

où $R^1$, $R^2$ et $R^3$ sont choisis, chacun indépendamment, dans l'ensemble des
groupes alkyle ramifiés ou non ramifiés ayant de 1 à 10 atomes de carbone ou représentent un atome d'hydrogène individuel,
pour l'amélioration de l'efficacité de substances actives lipophiles dans des préparations cosmétiques ou dermatologiques.

**2.** Utilisation selon la revendication 1, **caractérisée en ce que** les substances actives lipophiles sont choisies dans le groupe des substances actives qui ont une valeur log P qui est supérieure à 3,5.

**3.** Utilisation selon la revendication 1, **caractérisée en ce que** les substances actives lipophiles (un ou plusieurs composés) sont choisies dans les groupes des ubiquinones, rétinoïdes et/ou caroténoïdes.

**4.** Utilisation selon l'une quelconque des revendications précédentes, **caractérisée en ce que** la quantité de substances lipophiles (un ou plusieurs composés) dans les préparations est choisie dans la plage de 0,0001 à 1 % en poids, par rapport au poids total de la préparation.

**5.** Utilisation selon l'une quelconque des revendications précédentes, **caractérisée en ce que** la quantité de dérivés de bis-résorcinyltriazine (un ou plusieurs composés) dans les préparations est choisie dans la plage de 0,1 à 10 % en poids, par rapport au poids total de la préparation.

**6.** Utilisation selon l'une quelconque des revendications précédentes, **caractérisée en ce que** le rapport pondéral des dérivés de bis-résorcinyltriazine (un ou plusieurs composés) à la ou aux substance(s) active(s) lipophile(s) est choisi dans la plage de 1 000 : 1 à 1 : 10.

**7.** Utilisation selon l'une quelconque des revendications précédentes, **caractérisée en ce qu'**en tant que composés choisis dans le groupe des composés

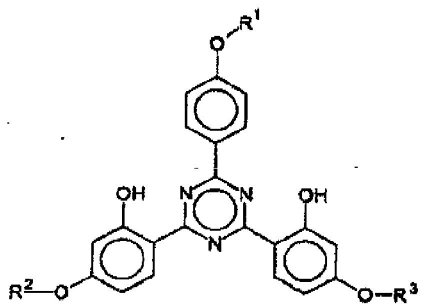

où $R^1$, $R^2$ et $R^3$ sont choisis, chacun indépendamment, dans l'ensemble des groupes alkyle ramifiés ou non ramifiés ayant de 1 à 10 atomes de carbone ou représentent un atome d'hydrogène individuel, on utilise un ou plusieurs composés choisis dans le groupe des composés sel sodique de 2,4-bis-{[4-(3-sulfonato)-2-hydroxy-propyloxy)-2-hydroxy]-phényl}-6-(4-méthoxyphényl)-1,3,5-triazine, la 2,4-bis-{[4-(3-(2-propyloxy)-2-hydroxypropyloxy)-2-hydroxy]-phényl}-6-(4-méthoxyphényl)-1,3,5-triazine, la 2,4-bis-{[4-(2-éthyl-hexyloxy)-2-hydroxy]-phényl}-6-[4-(2-méthoxyéthyl-carboxyl)-phénylamino]-1,3,5-triazine, la 2,4-bis-{[4-(3-(2-propyloxy)-2-hydroxy-propyloxy)-2-hydroxy]-phényl}-6-[4-(2-éthyl-carboxyl)-phénylamino]-1,3,5-triazine, la 2,4-bis-{[4-(2-éthyl-hexyloxy)-2-hydroxy]-phényl}-6-(1-méthylpyrrol-2-yl)-1,3,5-triazine, la 2,4-bis-{[4-tris(triméthylsiloxy-silylpropyloxy)-2-hydroxy]-phényl}-6-(4-méthoxyphényl)-1,3,5-triazine, la 2,4-bis-{[4-(2"-méthyl-propényloxy)-2-hydroxy]-phényl}-6-(4-méthoxyphényl)-1,3,5-triazine et la 2,4-bis-{[4-(1',1',1',3',5' 5',5'-heptaméthylsiloxy-2"-méthylpropyloxy)-2-hydroxy]-phényl}-6-(4-méthoxyphényl)-1,3,5-triazine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4144325 A
- US 4248861 A
- EP 775698 A
- WO 9220690 A


### In der Beschreibung aufgeführte Nicht-Patentliteratur


- **Schmutz.** *Chemikalien, Mikroorganismen*
- *Int. J. Cosm. Science,* 1988, vol. 10, 53
- **B. A. Voelckel et al.** *Zentralblatt Haut- und Geschlechtskrankheiten,* 1989, vol. 156, 2